# EUROPEAN PATENT APPLICATION

(11) **EP 3 366 225 A2**
(43) Date of publication of application: **29.08.2018**
(21) Application number: 18158826.0
(22) Date of filing: 27.02.2018
(51) Int. Cl.: A61B 17/00

(54) **REUSABLE POWERED SURGICAL DEVICES HAVING IMPROVED DURABILITY**

(30) Priority: 28.02.2017 US 201762464584 P; 22.01.2018 US 201815876378
(71) Applicant: Covidien LP, Mansfield, MA 02048 (US)
(72) Inventor: Mozdzierz, Patrick, Glastonbury, CT Connecticut 06033 (US)
(74) Representative: Maschio, Antonio

(57) **Abstract**

A surgical device includes at least one electronic component having a moisture collection agent sealed therein that is configured to protect electronic elements of the electronic component and/or a moisture mitigation system including a moisture migration component disposed within the surgical device and a moisture collection agent disposed adjacent to the moisture migration component to remove moisture from within the surgical device. The at least one electronic component may include a substrate, electronic elements disposed on a surface of the substrate, a coating covering the surface of the substrate and the electronic elements disposed thereon, and a moisture collection agent disposed between the substrate and the coating to control moisture at the surface of the substrate. The at least one electronic component may include a substrate having a base and a cover defining a sealed cavity therein, and electronic elements and a moisture collection agent disposed within the sealed cavity.

## Description

### CROSS-REFERENCE TO RELATED APPLICATIONS

This application claims the benefit of and priority to U.S. Provisional Patent Application Serial No. 62/464,584 filed February 28, 2017, the entire disclosure of which is incorporated by reference herein.

### TECHNICAL FIELD

The present disclosure relates generally to reusable surgical devices. More particularly, the present disclosure relates to powered surgical devices with enhanced durability and increased moisture resistance.

### BACKGROUND

Powered surgical devices include electronic components, such as printed circuit boards, switches, sensors, etc., to enhance the control of functions of the surgical devices. The intelligence of such surgical devices result in a higher product cost compared to currently available disposable units. Accordingly, it would be beneficial if such intelligent devices are reusable.

Reusable surgical devices must be cleaned (e.g., washed or disinfected using high pH solutions) and sterilized prior to subsequent uses. The most common method of sterilization is autoclaving, which utilizes high pressure superheated steam (e.g., 37 PSI @ 137°C for 18 minutes). Such environments, however, are known to damage various electronic components. Accordingly, the electronic components of reusable surgical devices may be protected from high temperatures, steam, and/or moisture by utilizing, for example, conformal coatings, potting, sealed enclosures, and/or overmolding.

The electronic components, however, may suffer from moisture ingress and/or release of residual moisture present in the materials (e.g., resin boards or plastics) of the electronic components during cleaning and/or autoclaving procedures which, in turn, may corrode and/or degrade the electronic components. Thus, it would be beneficial if the durability of the electronic components is enhanced to improve the reliability of the electronic components and/or extend the effective cycle life of the surgical devices.

### SUMMARY

The surgical devices of the present disclosure include electronic components having a moisture collection agent sealed with the circuitry thereof to control moisture within the electronic components. The electronic components are thus configured to withstand environmental stresses associated with high pH cleaning and sterilization (e.g., autowashing and/or autoclaving), minimizing and/or eliminating the ingress of fluids or release of residual moisture during such processes, thereby rendering the electronic components more durable for re-use.

The surgical devices of the present disclosure may additionally or alternatively include a moisture mitigation system including a moisture migration component and a moisture collection agent disposed within the surgical device or a component thereof, to remove moisture from within the surgical device.

In one aspect of the present disclosure, a surgical device includes at least one electronic component including a substrate, electronic elements disposed on a surface of the substrate, a coating covering at least a portion of the surface of the substrate and the electronic elements disposed thereon, and a moisture collection agent sealed between the substrate and the coating.

The at least one electronic component may be a circuit board.

In embodiments, the moisture collection agent is disposed in a layer positioned over the substrate and the electronic elements. The moisture collection agent may be dispersed in a polymer binder. In some embodiments, the moisture collection agent is disposed within at least one packet positioned on a portion of the substrate. In certain embodiments, the moisture collection agent is disposed between the coating and a film covering the substrate and the electronic elements. The moisture collection agent may be a desiccant.

The surgical device may further include a handle assembly, an adapter assembly, and/or an end effector in which the at least one electronic component is mounted.

In another aspect of the present disclosure, a surgical device includes at least one electronic component including a substrate having a base and a cover defining a sealed cavity therein, electronic elements disposed on a surface within the sealed cavity, and a moisture collection agent disposed within the sealed cavity.

The at least one electronic component may be a switch and/or a sensor.

In embodiments, the moisture collection agent is dispersed in a polymer binder, and in some embodiments, the moisture collection agent is disposed within a packet. The moisture collection agent may be disposed over the electronic elements or in spaced relation relative to the electronic components within the sealed cavity. The moisture collection agent may be a desiccant.

The surgical device may further include a handle assembly, an adapter assembly, and/or an end effector in which the at least one electronic component is mounted. In yet another aspect of the present disclosure, a surgical device includes a moisture mitigation system including a moisture migration component disposed within the surgical device and a moisture collection agent disposed adjacent to the moisture migration component. The moisture migration component draws moisture towards the moisture collection agent which, in turn, collects the moisture.

In embodiments, the moisture migration component is disposed adjacent to a moisture retention zone defined in the surgical device. In some embodiments, the moisture migration component is disposed adjacent to an electronic component of the surgical device.

The moisture migration component may be formed from a hydrophilic material, and/or the moisture collection agent may be a desiccant. In embodiments, the moisture collection agent is disposed within a cartridge.

The surgical device may further include a handle assembly, an adapter assembly, and/or an end effector in which the moisture mitigation system is disposed.

Other aspects, features, and advantages will be apparent from the description, drawings, and the claims.

### BRIEF DESCRIPTION OF THE DRAWINGS

Various aspects of the present disclosure are described herein below with reference to the drawings, which are incorporated in and constitute a part of this specification, wherein:
FIG. 1 is a perspective view of an electronic component in accordance with an embodiment of the present disclosure;
FIGS. 2A-2C are cross-sectional views of embodiments of the electronic component of FIG. 1, taken along line A-A of FIG. 1;
FIG. 3 is a perspective view of an electronic component in accordance with another embodiment of the present disclosure;
FIGS. 4A and 4B are cross-sectional views of embodiments of the electronic component of FIG. 3, taken along line B-B of FIG. 3;
FIG. 5 is a perspective view of a surgical device in accordance with an embodiment of the present disclosure;
FIG. 6A is a perspective view of an inner handle housing of a handle assembly of the surgical device of FIG. 5, with a housing shell of the inner handle housing separated therefrom;
FIG. 6B is a perspective view of an adapter assembly of the surgical device of FIG. 5;
FIG. 6C is a perspective view of an electronic assembly of the adapter assembly of FIG. 6B;
FIG. 6D is a perspective view of a distal portion of the adapter assembly of FIG. 6B, with an outer tube of the adapter assembly removed therefrom;
FIG. 7 is a perspective view of a surgical device in accordance with another embodiment of the present disclosure;
FIG. 8A is a perspective view of a distal portion of the adapter assembly of the surgical device of FIG. 7, with an outer sleeve of the adapter assembly removed therefrom;
FIG. 8B is a cross-sectional view of a force sensor of the adapter assembly of FIGS. 7 and 8A, taken along line C-C of FIG. 8A; and
FIG. 9 is a cross-sectional view of an outer knob housing and an outer tube of the adapter assembly of FIG. 5, with internal parts removed.

### DETAILED DESCRIPTION

Surgical devices in accordance with embodiments of the present disclosure include electronic component(s) that include a moisture collection agent sealed therein to protect the electronic component(s) from exposure to moisture during, for example, cleaning and/or sterilization procedures where the surgical devices may be subjected to high temperatures, steam, chemicals, and/or moisture.

Surgical devices in accordance with embodiments of the present disclosure include moisture mitigation system(s) that include a moisture migration component configured to draw moisture away from moisture retention zone(s) of the surgical devices and toward a moisture collection agent to extract moisture which may accumulate or be retained within the surgical device after, for example, cleaning and/or sterilization procedures.

Embodiments of the present disclosure are now described in detail with reference to the drawings in which like reference numerals designate identical or corresponding elements in each of the several views. Throughout this description, the term "proximal" refers to a portion of a device, or component thereof, that is closer to a hand of a user, and the term "distal" refers to a portion of the device, or component thereof, that is farther from the hand of the user.

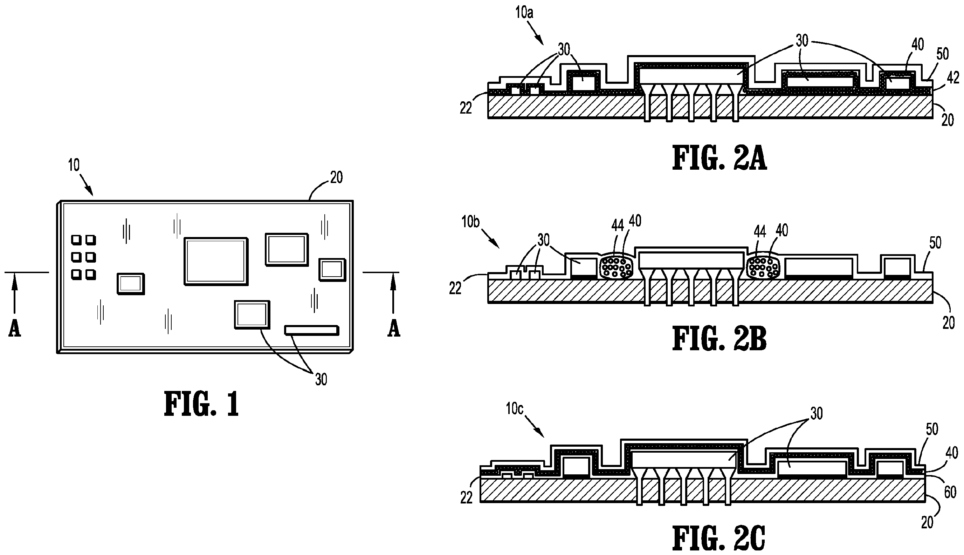

Turning now to FIG. 1, an electronic component 10, in accordance with an embodiment of the present disclosure, is shown in the form of a circuit board. It should be understood that while the electronic component 10 is shown and described as a circuit board, the electronic component 10 may be any electronic component including circuitry disposed on a substrate and having a coating protecting the circuitry, as is within the purview of those skilled in the art.

As shown in FIGS. 2A-2C, in conjunction with FIG. 1, embodiments of the electronic component 10 (designated as electronic components 10a-10c, respectively) include a substrate 20, electronic elements 30 mounted on a surface 22 of the substrate 20, a moisture collection agent 40 disposed over the substrate 20 and/or the electronic elements 30, and a coating 50 disposed thereover to seal the electronic elements 30 and the moisture collection agent 40 between the substrate 20 and the coating 50.

The substrate 20 may be formed from any material suitable for supporting and electrically connecting the electronic elements 30. In embodiments, the substrate 20 is formed from one or more layers or sheets of dielectric material, such as a polymer or a ceramic. In embodiments, the substrate 20 is a printed circuit board.

The electronic elements 30 may be surface mount technology and/or through-hole technology, including, for example, integrated circuits or microchips, resistors, inductors, capacitors, sensing elements (e.g., optical sensors, pressure sensors, and capacitive sensors), buttons, switches, electrical connectors, wires, and/or traces, among other circuitry within the purview of those skilled in the art.

The moisture collection agent 40 may be any moisture absorbing or adsorbing material that aids in controlling the humidity or moisture level at the surface 22 of the substrate 20 to minimize corrosion and/or degradation of the electronic elements 30 (e.g., maintaining a humidity level within the electronic component 10 that is equal to or lower than a humidity level at which the electronic elements 30 are sensitive). In embodiments, the moisture collection agent 40 is a desiccant.

The coating 50 is disposed over the substrate 20, the electronic elements 30, and the moisture collection agent 40. The coating 50 may be any protective layer that seals the electronic elements 30 and the moisture collection agent 40 to the substrate 20 thereby protecting the electronic elements 30 from the outside environment. In embodiments, the coating 50 is a conformal layer that conforms to the topography of the substrate 20 on which the electronic elements 30 and the moisture collection agent 40 are disposed. It should be understood that the coating 50 may cover a portion of the surface 22 of the substrate 20 (e.g., portions on which the electronic elements 30 are disposed) or an entirety of the surface 22 of the substrate 20.

The moisture collection agent 40 may be disposed between the surface 22 of the substrate 20 and the coating 50 of the electronic component 10 in a variety of configurations depending on, for example, the type of moisture collection agent 40 utilized within the electronic component 10 and/or the sensitivity of the electronic elements 30 mounted thereon to moisture.

As shown in FIG. 2A, the moisture collection agent 40 is provided as a continuous uniform layer 42 (e.g., the moisture collection agent 40 may be dispersed in a polymer binder to form a sheet) disposed over the substrate 20 and the electronic elements 30, with the coating 50 disposed over the layer 42 of the moisture collection agent 40. It should be understood, however, that the layer 42 of moisture collection agent 40 may be a partial layer, or a plurality of layers, disposed on one or more portions or an entirety of the substrate 20 and/or electronic elements 30. As shown in FIG. 2B, the moisture collection agent 40 is provided on portions of the substrate 20 and/or electronic elements 30, and is retained within one or more discrete packets 44 of moisture collection agent 40. The packets 44 may include the same or different amounts and/or types of moisture collection agent 40. As shown in FIG. 2C, the moisture collection agent 40 is disposed between the coating 50 and a film 60 disposed over the substrate 20 and the electronic elements 30. The coating and the film 50, 60 may be the same or different.

In embodiments, an electronic component 10 is sealed for use by fully drying and assembling the electronic component 10 in an inert gas environment, such as an inert glove box (e.g., a nitrogen-filled atmosphere), to ensure zero moisture content within the electronic component 10. Accordingly, in the event that the seal should fail and moisture should permeate or ingress through the coating 50, the moisture collection agent 40 will absorb or adsorb the moisture that may otherwise attack the substrate 20 and/or electronic elements 30 of the electronic component 10. It should be understood that the moisture collection agent 40 may also collect any moisture which may be trapped within the electronic component 10, such as moisture which may have been introduced into the electronic component 10 during assembly and/or residual moisture present in the materials of the electronic component 10.

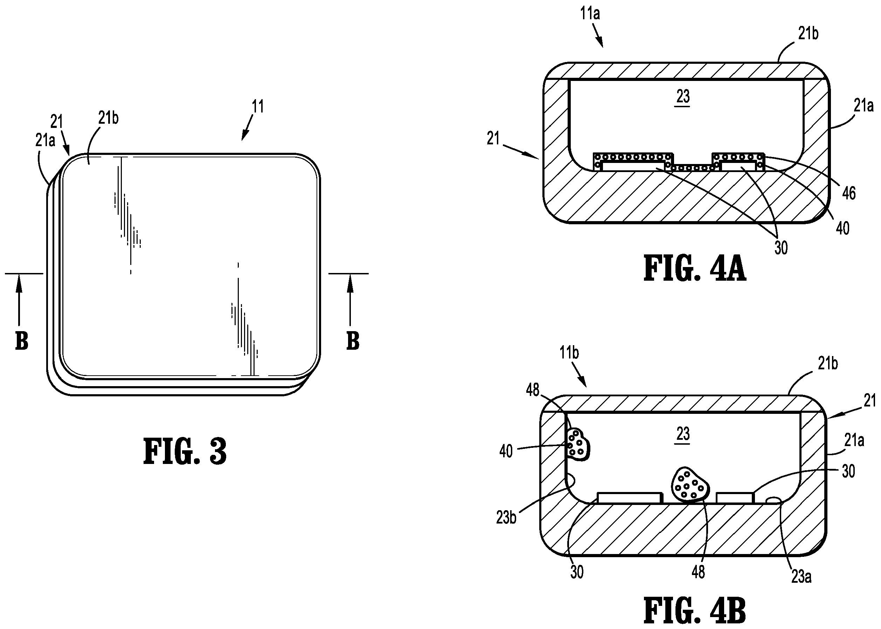

Referring now to FIG. 3, an electronic component 11, in accordance with another embodiment of the present disclosure, is shown as a sealed component. It should be understood that the electronic component 11 may be any electronic component including circuitry disposed within a sealed cavity or chamber, such as sensors, switches, power sources, etc., as is within the purview of those skilled in the art.

As shown in FIGS. 4A and 4B, in conjunction with FIG. 3, embodiments of the electronic component 11 (designated as electronic components 11a, 11b, respectively) include a substrate 21 having a base 21a and a cover 21b that together define a sealed cavity 23 (e.g., a hermetically sealed cavity) therein. The electronic elements 30 are mounted to the substrate 21 within the cavity 23, and the moisture collection agent 40 is disposed within the cavity 23 such that the electronic elements 30 and the moisture collection agent 40 are positioned within the cavity 23 in a fluid tight manner.

The moisture collection agent 40 may be disposed within any portion of the cavity 23 to aid in controlling moisture within the cavity 23. As discussed above with regard to electronic component 10, should the seal fail and moisture should permeate or ingress into the electronic component 11 (e.g., be introduced into the cavity 23 through the connection point between the base 21a and the cover 21b), the moisture collection agent 40 will absorb or adsorb the moisture, as well as any moisture that may be trapped within the electronic component 11 upon assembly and/or released from the materials of the electronic component 11.

As shown in FIG. 4A, the moisture collection agent 40 is disposed over the electronic elements 30 as a layer 46 of moisture collection agent 40. The layer 46 may be continuous or discontinuous as discussed above, for example, with regard to layer 42. As shown in FIG. 4B, the moisture collection agent 40 is retained within one or more discrete packets 48 of moisture collection agent 40, which may be disposed adjacent to the electronic elements 30, such as on a surface 23a of the cavity 23 on which the electronic elements 30 are mounted, or in spaced relation relative thereto, such as on a wall 23b of the cavity 23.

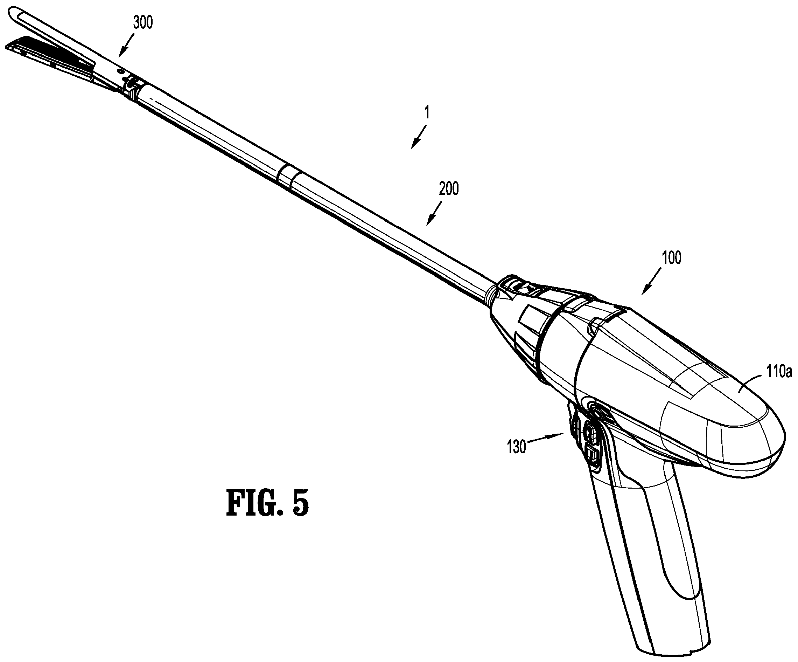

It should be understood that electronic components disposed within powered surgical devices may be configured as shown and described above with regard to electronic components 10, 11. For example, turning now to FIG. 5, a surgical device 1 in accordance with an embodiment of the present disclosure, is in the form of a powered handheld electromechanical surgical instrument, and includes a powered handle assembly 100, an adapter assembly 200, and a tool assembly or end effector 300. The powered handle assembly 100 is configured for selective connection with the adapter assembly 200 and, in turn, the adapter assembly 200 is configured for selective connection with the end effector 300.

The surgical device 1 will only further be described to the extent necessary to disclose aspects of the present disclosure. For a detailed description of the structure and function of exemplary surgical devices, reference may be made to commonly owned U.S. Patent Appl. Serial Nos. 14/991,157, filed on January 8, 2016, and 15/096,399, filed on April 12, 2016, the entire contents of each of which are incorporated herein by reference.

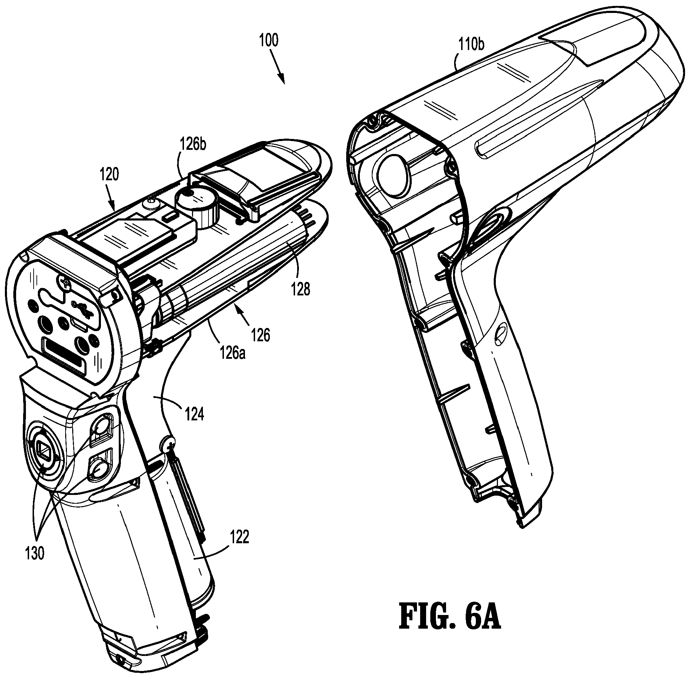

With reference to FIG. 6A, in conjunction with FIG. 5, the handle assembly 100 includes an inner handle housing 110b disposed within a housing shell 110a (FIG. 5). The inner handle housing 110b houses a power-pack 120 configured to power and control various operations of the surgical device 1, and a plurality of actuators 130 (e.g., finger-actuated control buttons, knobs, toggles, slides, interfaces, and the like) for activating various functions of the surgical device 1.

The power-pack 120 includes a rechargeable battery 122 configured to supply power to any of the electrical components of surgical device 100, a battery circuit board 124, and a controller circuit board 126. The controller circuit board 126 includes a motor controller circuit board 126a electrically connected with a main controller circuit board 126b (e.g., via a ribbon cable (not shown)). The motor controller circuit board 126a is communicatively coupled with the battery circuit board 124 enabling communication therebetween and between the battery circuit board 124 and the main controller circuit board 126b. A plurality of motors 128 are disposed between, and electrically connected to, the controller circuit board 126 and the battery 122 to, for example, drive functions of the end effector 300 (FIG. 5).

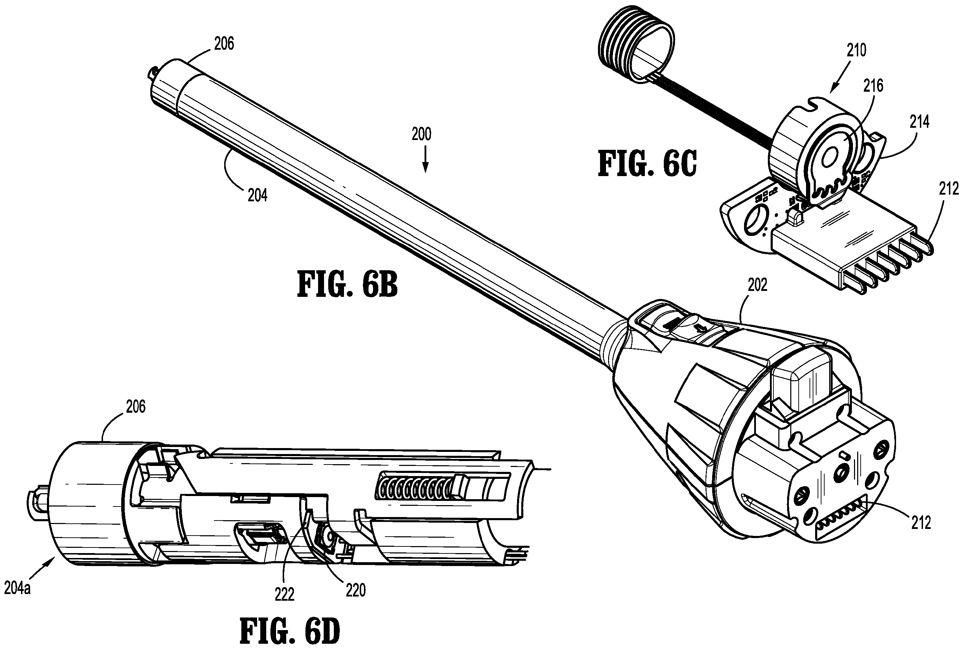

With reference now to FIG. 6B, the adapter assembly 200 includes an outer knob housing 202 and an outer tube or sleeve 204 extending from a distal end of the outer knob housing 202 and terminating at a distal cap 206. As shown in FIG. 6C, in conjunction with FIG. 6B, an electrical assembly 210 is supported on and in outer knob housing 202. The electrical assembly 210 includes a plurality of electrical contact blades 212 supported on a circuit board 214 for electrical connection to the handle assembly 100 (FIG. 5) of the surgical device 1, and a strain gauge 216 electrically connected to the circuit board 214 for closed-loop feedback of firing/clamping loads exhibited by the adapter assembly 200 and regulated by the power-pack 120 (FIG. 6A). The electrical assembly 210 serves to allow for calibration and communication information (i.e., identifying information, life-cycle information, system information, force information) to the main controller circuit board 126b (FIG. 6A) of the power-pack 120 of the handle assembly 100.

As shown in FIG. 6D, a switch 220 is disposed within the outer tube 204 (FIG. 6B) of the adapter assembly 200. The switch 220 is configured to toggle in response to a coupling of the end effector 300 (FIG. 5) to a distal end 204a of the outer tube 204. The switch 220 is mounted on a printed circuit board 222 that is electrically connected with the controller circuit board 126 (FIG. 6A) of the power-pack 120. The switch 220 is configured to couple to a memory (not shown) of the end effector 300 which, in turn, is configured to store data pertaining to the end effector 300 and is configured to provide the data to the controller circuit board 126 of the handle assembly 100 in response to the end effector 300 being coupled to the outer tube 204.

The circuit boards (e.g., the battery circuit board 124, the controller circuit board 126, including the motor controller circuit board 126a and the main controller circuit board 126b, the circuit board 214 of the electrical assembly 210, and the printed circuit board 222), the actuators 130, the strain gauge 216, and/or the switch 220 of the handle and adapter assemblies 100, 200 of the surgical device 1 may be configured as shown and described, for example, with regard to the electronic components 10, 11.

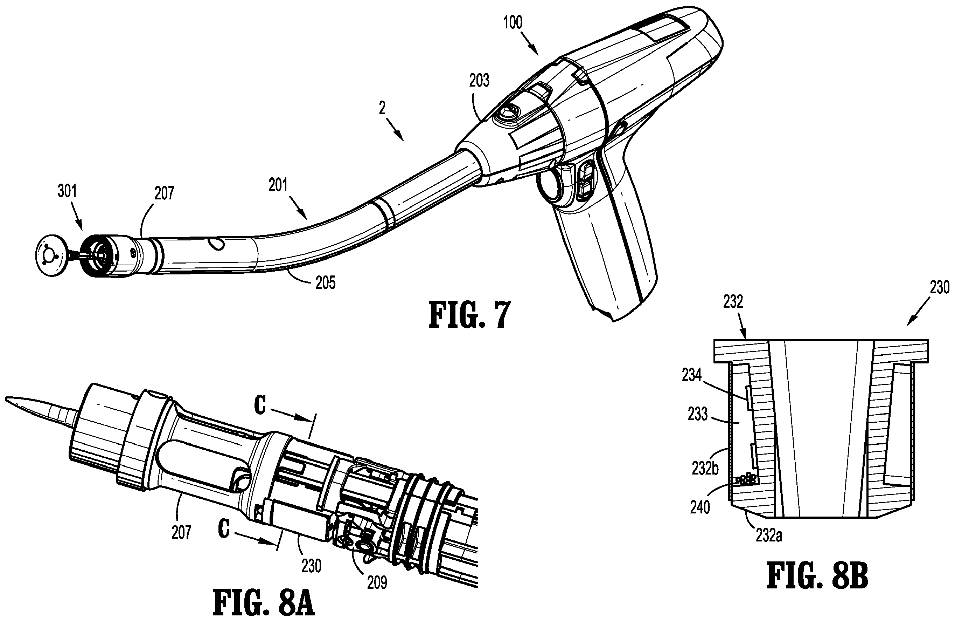

As another example, shown in FIG. 7, a surgical device 2 in accordance with another embodiment of the present disclosure includes the powered handle assembly 100, an adapter assembly 201, and an end effector 301. It should be understood that a variety of different adapter assemblies and end effectors may be utilized with the handle assembly 100 of the present disclosure. For a detailed description of the structure and function of exemplary adapter assemblies, reference may be made to commonly owned U.S. Provisional Patent Appl. Serial No. 62/251,930, filed on November 6, 2015, the entire contents of which are incorporated herein by reference.

The adapter assembly 201 includes an outer knob housing 203 and an outer sleeve 205. A distal connector housing 207 is secured to a distal end of the outer sleeve 205, and is configured to releasably secure the end effector 301 to the adapter assembly 201. As shown in FIG. 8A, the adapter assembly 201 includes a force sensor 230 disposed between the distal connector housing 207 and a trocar connection housing 209 of the adapter assembly 200. The force sensor 230 is configured to measure forces along a load path from the end effector 301 against the distal connector housing 207 during clamping and/or stapling of tissue. For a detailed description of exemplary force sensors, reference may be made to U.S. Provisional Patent Appl. Serial No. 62/375,043, filed on August 15, 2016, the entire contents of which are incorporated herein by reference.

The force sensor 230 of the adapter assembly 201 may be configured as shown and described, for example, with regard to the electronic components 10, 11. For example, as shown in FIG. 8B, the force sensor 230 includes a substrate 232 having a base 232a and a cover 232b in the form of a plate that is secured (e.g., welded) to the base 232a to define a hermetically sealed cavity 233 therein. Electronic elements 234 (e.g., sensing elements) are disposed within the cavity 233, and a moisture collection agent 240 is also disposed within the cavity 233 to absorb or adsorb moisture which may enter the cavity 233 or be released from the substrate 232.

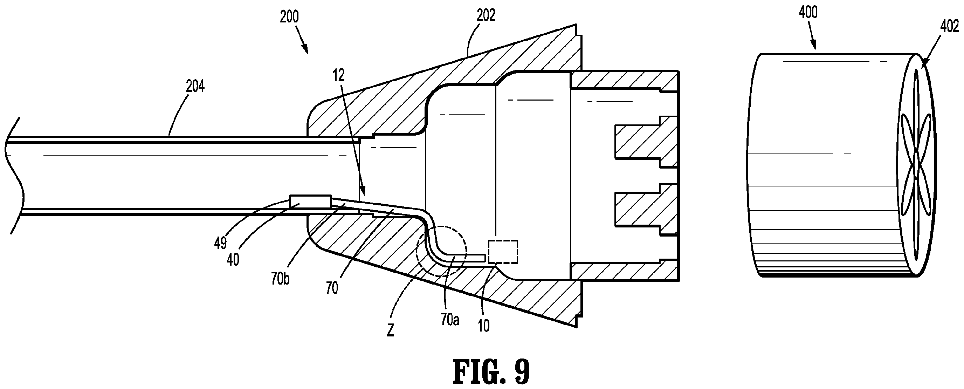

Turning now to FIG. 9, a moisture mitigation system 12 in accordance with an embodiment of the present disclosure is shown. The moisture mitigation system 12 may be utilized alone or in combination with electronic component(s) 10, 11 of the present disclosure, to reduce or remove moisture from within a surgical device or a component thereof. Surgical devices may include, for example, moisture retention zone(s) "Z" which are areas in which moisture (e.g., from cleaning, autoclaving, etc.) is likely to pool and/or areas in which moisture is difficult to evacuate (e.g., during a vacuum cycle following an autoclave procedure). The moisture retention zones include, for example, surfaces within the surgical device which are flat or lay horizontal, as well as crevices, passageways, joints, among other areas susceptible to collecting moisture as is within the purview of those skilled in the art.

The moisture mitigation system 12 includes a moisture migration component 70 and a moisture collection agent 40. The moisture migration component 70 includes a first end portion 70a disposed adjacent to or within a moisture retention zone "Z" (e.g., a bend in the outer knob housing 202 of an adapter assembly 200) and a second end portion 70b disposed adjacent to or within the moisture collection agent 40. The moisture migration component 70 is configured to wick (e.g., via capillary action) or otherwise draw moisture away from the moisture retention zone "Z" and towards (e.g., into) the moisture collection agent 40. In embodiments, the moisture migration component 70 is a hydrophilic material. In some embodiments, the moisture migration component 70 may be configured to both draw moisture towards the moisture collection agent 40 and absorb moisture until the moisture can be evaporated therefrom (e.g., by heating).

In embodiments, the moisture migration component 70 may be disposed adjacent to an electronic component 10 (shown in phantom in FIG. 9) of a surgical device to aid in preventing moisture ingress into the electronic component 10.

The moisture collection agent 40 may be provided in a variety of forms as described above with regard to the electronic components 10, 11. In some embodiments, the moisture collection agent 40 may be provided as a replaceable cartridge 49 so that the moisture collection agent 40 may be replaced as needed (e.g., the cartridge may be configured for single or multiple use depending on, for example, the type and/or amount of moisture collection agent 40 used).

The moisture collection agent 40 may be reusable (e.g., the moisture can be evacuated therefrom such that the moisture collection agent 40 may be used for subsequent cycles). Moisture from the moisture collection agent 40 may be evacuated by, for example, performing a vacuum cycle (e.g., after autoclave) or subjecting the surgical device or component thereof containing the moisture collection agent 40 to a drying cycle using, for example, a vacuum or a blower, with or without heat, to evaporate the moisture.

The moisture collection agent 40 may be color changing so that a user can visually inspect the surgical device or component thereof to see when to replace or reprocess (e.g., bake, heat, or dry) the moisture collection agent 40.

In embodiments, as shown in FIG. 9, for example, a blower 400 may be utilized with the adapter assembly 200 to ensure moisture evacuation from the moisture collection agent 40. The blower 400 includes a fan 402, and may be connectable (e.g., docked) to the adapter assembly 200 after cleaning and/or sterilization. Alternatively, the blower 400 may be integrated into the adapter assembly 200.

It should be understood that while the moisture mitigation system 12 is shown and described above as being disposed within an adapter assembly 200 of a surgical device 1, the moisture mitigation system 12 may be utilized in the surgical devices 1, 2 as described above, or in components thereof, in one or more areas identified as moisture retention zones. It should also be understood that while the moisture mitigation system 12 is described and shown disposed within a powered surgical device, the moisture mitigation system 12 may be utilized in non-powered surgical devices (e.g., reusable surgical devices subject to washing and/or sterilization procedures).

Persons skilled in the art will understand that the structures specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. For example, the electronic components and/or moisture mitigation system of the present disclosure may be utilized in other surgical devices, such as robotic or powered surgical devices/instruments that are subject to sterilization procedures (e.g., autoclaving and/or autowashing). Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

The invention may be described by reference to the following numbered paragraphs:-
1. A surgical device including at least one of a handle assembly, an adapter assembly, or an end effector, the surgical device comprising:
   at least one electronic component mounted within the at least one of the handle assembly, the adapter assembly, or the end effector of the surgical device, the at least one electronic component including:
      a substrate;
      electronic elements disposed on a surface of the substrate;
      a coating covering at least a portion of the surface of the substrate and the electronic elements disposed thereon; and
      a moisture collection agent sealed between the substrate and the coating.
2. The surgical device according to paragraph 1, wherein the at least one electronic component is a circuit board.
3. The surgical device according to paragraph 1, wherein the moisture collection agent is a desiccant.
4. The surgical device according to paragraph 1, wherein the moisture collection agent is disposed in a layer positioned over the substrate and the electronic elements.
5. The surgical device according to paragraph 1, wherein the moisture collection agent is dispersed in a polymer binder.
6. The surgical device according to paragraph 1, wherein the moisture collection agent is disposed within at least one packet positioned on a portion of the substrate.
7. The surgical device according to paragraph 1, where the moisture collection agent is disposed between the coating and a film covering the substrate and the electronic elements.
8. A surgical device including at least one of a handle assembly, an adapter assembly, or an end effector, the surgical device comprising:
   at least one electronic component mounted within the at least one of the handle assembly, the adapter assembly, or the end effector of the surgical device, the at least one electronic component including:
      a substrate having a base and a cover defining a sealed cavity therein;
      electronic elements disposed on a surface within the sealed cavity; and
      a moisture collection agent disposed within the sealed cavity.
9. The surgical device according to paragraph 8, wherein the at least one electronic component is a switch.
10. The surgical device according to paragraph 8, wherein the at least one electronic component is a sensor.
11. The surgical device according to paragraph 8, wherein the moisture collection agent is a desiccant.
12. The surgical device according to paragraph 8, wherein the moisture collection agent is dispersed in a polymer binder.
13. The surgical device according to paragraph 8, wherein the moisture collection agent is disposed within a packet.
14. The surgical device according to paragraph 8, wherein the moisture collection agent is disposed over the electronic elements.
15. The surgical device according to paragraph 8, wherein the moisture collection agent is disposed within the sealed cavity in spaced relation relative to the electronic components.
16. A surgical device including at least one of a handle assembly, an adapter assembly, or an end effector, the surgical device comprising:
   a moisture mitigation system including:
      a moisture migration component disposed within the at least one of the handle assembly, the adapter assembly, or the end effector of the surgical device; and
      a moisture collection agent disposed adjacent to the moisture migration component,
   wherein the moisture migration component draws moisture towards the moisture collection agent which, in turn, collects the moisture.
17. The surgical device according to paragraph 16, wherein the moisture migration component is disposed adjacent to a moisture retention zone defined in the surgical device.
18. The surgical device according to paragraph 16, wherein the moisture migration component is disposed adjacent to an electronic component of the surgical device.
19. The surgical device according to paragraph 16, wherein the moisture migration component is formed from a hydrophilic material.
20. The surgical device according to paragraph 16, wherein the moisture collection agent is a desiccant.

## Claims

1. A surgical device including at least one of a handle assembly, an adapter assembly, or an end effector, the surgical device comprising:
at least one electronic component mounted within the at least one of the handle assembly, the adapter assembly, or the end effector of the surgical device, the at least one electronic component including:
a substrate;
electronic elements disposed on a surface of the substrate;
a coating covering at least a portion of the surface of the substrate and the electronic elements disposed thereon; and
a moisture collection agent sealed between the substrate and the coating.

2. The surgical device according to claim 1, wherein the at least one electronic component is a circuit board.

3. The surgical device according to claim 1 or claim 2, wherein the moisture collection agent is a desiccant; and/or wherein the moisture collection agent is disposed in a layer positioned over the substrate and the electronic elements.

4. The surgical device according to any preceding claim, wherein the moisture collection agent is dispersed in a polymer binder.

5. The surgical device according to any preceding claim, wherein the moisture collection agent is disposed within at least one packet positioned on a portion of the substrate or where the moisture collection agent is disposed between the coating and a film covering the substrate and the electronic elements.

6. A surgical device including at least one of a handle assembly, an adapter assembly, or an end effector, the surgical device comprising:
at least one electronic component mounted within the at least one of the handle assembly, the adapter assembly, or the end effector of the surgical device, the at least one electronic component including:
a substrate having a base and a cover defining a sealed cavity therein;
electronic elements disposed on a surface within the sealed cavity; and
a moisture collection agent disposed within the sealed cavity.

7. The surgical device according to claim 6, wherein the at least one electronic component is a switch and/or wherein the at least one electronic component is a sensor.

8. The surgical device according to claim 6 or claim 7, wherein the moisture collection agent is a desiccant.

9. The surgical device according to any of claims 6 to 8, wherein the moisture collection agent is dispersed in a polymer binder; and/or wherein the moisture collection agent is disposed within a packet; and/or wherein the moisture collection agent is disposed over the electronic elements.

10. The surgical device according to any of claims 6 to 9, wherein the moisture collection agent is disposed within the sealed cavity in spaced relation relative to the electronic components.

11. A surgical device including at least one of a handle assembly, an adapter assembly, or an end effector, the surgical device comprising:
a moisture mitigation system including:
a moisture migration component disposed within the at least one of the handle assembly, the adapter assembly, or the end effector of the surgical device; and
a moisture collection agent disposed adjacent to the moisture migration component,
wherein the moisture migration component draws moisture towards the moisture collection agent which, in turn, collects the moisture.

12. The surgical device according to claim 11, wherein the moisture migration component is disposed adjacent to a moisture retention zone defined in the surgical device; and/or wherein the moisture migration component is disposed adjacent to an electronic component of the surgical device.

13. The surgical device according to claim 11 or claim 12, wherein the moisture migration component is formed from a hydrophilic material.

14. The surgical device according to any of claims 11 to 13, wherein the moisture collection agent is a desiccant.
